(19) Europäisches Patentamt
European Patent Office
Office européen des brevets

(11) Veröffentlichungsnummer: **0 078 991**
**B1**

# (12) EUROPÄISCHE PATENTSCHRIFT

(45) Veröffentlichungstag der Patentschrift: **27.12.85**

(51) Int. Cl.⁴: **C 07 D 251/34**, C 08 G 18/72

(21) Anmeldenummer: **82109959.5**

(22) Anmeldetag: **28.10.82**

(54) **Verfahren zur Herstellung von Mischtrimerisaten organischer Isocyanate, die nach dem Verfahren erhaltenen Mischtrimerisate, sowie ihre Verwendung zur Herstellung von Polyurethanen.**

(30) Priorität: **10.11.81 DE 3144672**

(43) Veröffentlichungstag der Anmeldung: **18.05.83 Patentblatt 83/20**

(45) Bekanntmachung des Hinweises auf die Patenterteilung: **27.12.85 Patentblatt 85/52**

(84) Benannte Vertragsstaaten: **BE DE FR GB IT NL**

(56) Entgegenhaltungen:
**EP - A - 0 047 452**
**GB - A - 1 166 316**

**CHEMICAL ABSTRACTS, Band 94, 1981, Seite 83, Nr. 158365y, Columbus, Ohio, US, E.G. CHEREDNIKOVA u.a.: "Synthesis and study of polyisocyanates of isocyanurate structure"**

(73) Patentinhaber: **BAYER AG, Konzernverwaltung RP Patentabteilung, D-5090 Leverkusen 1 Bayerwerk (DE)**

(72) Erfinder: **Richter, Roland, Dr., Roggendorfstrasse 53, D-5000 Köln 80 (DE)**
Erfinder: **Müller, Hanns Peter, Dr., Im Kerberich 6, D-5068 Odenthal (DE)**
Erfinder: **Wagner, Kuno, Dr., Am Kiesberg 8, D-5090 Leverkusen (DE)**
Erfinder: **Riberi, Bernd, Dr., Jakob-Böhmestrasse 2, D-5000 Köln 80 (DE)**
Erfinder: **Fröhlich, Jürg, Dr., Paffenlöh 36, D-5093 Burscheid 1 (DE)**

**EP 0 078 991 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von Mischtrimerisaten aus organischen Isocyanaten einer, im Sinne der Isocyanattrimerisierungsreaktion unterschiedlichen Reaktivität, die nach dem Verfahren erhaltenen Mischtrimerisate und die Verwendung der nach dem Verfahren erhaltenen, freie Isocyanatgruppen aufweisenden Mischtrimerisate, gegebenenfalls in mit Blockierungsmittel für Isocyanatgruppen blockierter Form, bei der Herstellung von Polyurethankunststoffen.

Verfahren zur Trimerisierung organischer Isocyanate sind bekannt (vgl. z. B. G. A. Mills, Advances in Catalysis, Vol. 13, 393 (1962), J. H. Saunders, K. C. Frisch, Polyurethanes, Chemistry and Technology, Interscience, New York (1962); J. E. Kresta, K. H. Hsieh, Org. Coat. Plast. Chem. 42, 516 (1980); die DE-OS 1 201 992, 1 954 093, 2 325 826, 2 551 634, 2 641 380, 2 644 684, 2 726 749 und 2 901 479; GB-PS 949 253; die EP-OS 9 694 und 10 589; US-PS 3 622 577 oder die deutschen Patentanmeldungen 3 100 262.5 bzw. 3 100 263.3).

Die Herstellung von Mischtrimerisaten aromatischer und aliphatischer Diisocyanate wird beispielsweise in der DE-PS 1 670 667 beschrieben. Hierzu werden beispielsweise Gemische aus Hexamethylen-diisocyanat und 2,4-Diisocyanat-toluol in Gegenwart von organischen Phosphinen als Trimerisierungskatalysator teiltrimerisiert, wobei allerdings das aliphatische Diisocyanat in weit geringerem Umfang, als seiner Konzentration im Ausgangsgemisch entspricht, in das Trimerisat eingebaut wird. Die Mitverwendung der aliphatischen Isocyanate beim Verfahren der genannten Vorveröffentlichung erfolgt daher weniger mit dem Ziel der Herstellung von Mischtrimerisaten als vielmehr wegen des cokatalytischen Einflusses des aliphatischen Diisocyanats auf die Phosphin-katalysierte Trimerisierung von aromatischen Diisocyanaten. Trotz dieser cokatalytischen Wirkung des aliphatischen Diisocyanats liegen in den Reaktionsgemischen der Vorveröffentlichung nach der Abstoppung der Trimierisierungsreaktion zwecks Herstellung von Isocyanatgruppen-aufweisenden Trimerisaten stets noch beträchtliche Mengen an nicht trimerisierten aromatischen Ausgangsdiisocyanaten vor, so daß bei der Rückgewinnung der nicht umgesetzten Diisocyanate eine Auftrennung in aliphatisches und aromatisches Ausgangsdiisocyanat erforderlich ist. Ein weiterer Nachteil des Verfahrens der DE-PS 1 670 667 ist in der zwingend erforderlichen Verwendung von organischen Phosphinen als Trimerisierungskatalysatoren zu sehen. Da organische Phosphine in erster Linie Dimerisierungskatalysatoren für organische Diisocyanate darstellen, verläuft die Trimerisierungsreaktion vermutlich auch beim Verfahren der DE-PS 1 670 667 über die Zwischenstufe von Dimeren (Uretdionen). Nur aufgrund dieses speziellen Reaktionsmechanismus kann im übrigen die Bildung von Mischtrimerisaten beim Verfahren der Vorveröffentlichung erklärt werden. Bei der Trimerisierung von Gemischen von Isocyanaten einer unterschiedlichen Reaktivität mit anderen Trimerisierungskatalysatoren, die keine Uretdione bilden, entstehen nämlich vorwiegend Homotrimerisate der aromatischen Diisocyanate, da die reaktionsträgeren aliphatischen Diisocyanate erst nach Erreichen eines gewissen Verdünnungsgrades der aromatischen Diisocyanate an der Trimerisierungsreaktion teilnehmen und dann nur in untergeordnetem Umfang Mischtrimerisate, vorzugsweise jedoch Homotrimerisate bilden, so daß neben aromatischen und aliphatischen Homotrimerisaten nur untergeordnete Mengen an echten Mischtrimerisaten vorliegen. Echte Mischtrimerisate bei der Trimerisierung von Isocyanatgemischen entstehen nur, wenn man Gemische von Isocyanaten mit chemisch weitgehend äquivalenten NCO-Gruppen trimerisiert. So kann man z. B. die beiden aromatischen Monoisocyanate 4-Chlorphenylisocyanat und 3,4-Dichlorphenylisocyanat mit geeigneten Katalysatoren mischtrimerisieren, wobei der Anteil an Mischtrimerisat allein durch die Gemischzusammensetzung bestimmt wird.

Kürzlich wurde von S. Dabi und A. Zilkha in European Polymer Journal Vol. 17, pp. 35 (1981) für die Mischtrimerisierung weniger ausgewählter Mono- und Diisocyanate ein spezieller Katalysatortyp, Tributylzinnoxid, beschrieben, jedoch verlangt das empfohlene Verfahren Temperaturen, die über 100°C liegen und einen Katalysatoranteil, der größer als 1 Gew.-% ist, wodurch die Qualität der entstehenden Verfahrensprodukte stark beeinträchtigt und eine technische Verwendung, insbesondere für den Polyurethan-Lacksektor, ausgeschlossen wird. Außerdem bilden sich auch mit diesem Katalysatortyp Uretdione als Nebenprodukte.

Aufgabe der vorliegenden Erfindung war es, ein allgemeines Herstellungsverfahren zur Verfügung zu stellen, nach welchem man aus beliebigen Gemischen unterschiedlich reaktiver Isocyanate in einem Schritt mit einem Katalysator als Hauptprodukt echte Mischtrimerisate erhält, wobei die Verfahrensbedingungen denen des Standes der Technik zur Homotrimerisation von Polyisocyanaten zu Isocyanuratgruppen aufweisenden Polyisocyanaten ähnlich sein sollten, da die erhaltenen Verfahrensprodukte bevorzugt bei der Herstellung von Polyurethan-Kunststoffen Verwendung finden. Eine technische Verwendungsmöglichkeit der Isocyanatgruppen aufweisenden Mischtrimerisate ist aber nur dann gegeben, wenn der Katalysatoranteil so klein ist, daß er leicht desaktiviert und gegebenenfalls abgetrennt werden kann, wenn die thermische Belastung so gering ist, daß keine nachteiligen Verfärbungen der Verfahrensprodukte hervorgerufen werden und wenn keine Uretdione als Nebenprodukte gebildet werden, da aus diesen bei Lagerung infolge Rückspaltung die toxikologisch bedenklichen monomeren Isocyanate entstehen können.

Überraschenderweise wurde nunmehr gefunden, daß die Lösung diese Aufgabe mit dem nachstehenden näher beschriebenen erfindungsgemäßen Verfahren gelingt, bei welchem man das reaktionsträgere Isocyanat im Überschuß vorlegt, mit einer für dessen Trimerisierung ausreichenden Menge Katalysator versetzt, die Lösung kurzzeitig antrimerisiert und dann das reaktivere Isocyanat zu dem aktivierten reaktionsträgeren Isocyanat kontinuierlich hinzugibt. Beim erfindungsgemäßen Verfahren werden, bezogen auf die Gesamtmenge der gebildeten Trimerisate mehr als 50%, vorzugsweise mehr als 70% an echten Mischtrimerisaten gebildet, wobei der Anteil der im Mischtrimerisat eingebauten Isocyanate niedrigerer bzw. höherer Reaktivität auf einfache Weise durch die Zugabegeschwindigkeit der Isocyanatkomponente höherer Reaktivität und durch das Gesamtverhältnis (Isocyanatkomponente niedrigerer Reaktivität) : (Isocyanatkomponente höherer Reaktivität) gesteuert werden kann.

Gegenstand der Erfindung ist somit ein Verfahren zur Herstellung von Mischtrimerisaten durch zumindest teilweise Trimerisierung der Isocyanatgruppen von mindestens zwei organischen Isocyanaten einer im Sinne der Trimerisierungsreaktion unterschiedlicher Reaktivität in Gegenwart von, die Trimerisierung von Isocyanatgruppen unter Isocyanuratbildung beschleunigenden, Katalysatoren und gegebenenfalls Abbruch der Trimerisierungsreaktion beim jeweils erwünschten Trimerisierungsgrad durch thermische Zersetzung des Trimerisierungskatalysators und/oder durch Zugabe eines Katalysatorengiftes, dadurch gekennzeichnet, daß man die weniger reaktive Isocyanatkomponente vorlegt, anschließend in Gegenwart des Katalysators unter Trimerisierung von mindestens 0,1% der Isocyanatgruppen anpolymerisiert und schließlich zu dem so erhaltenen Reaktionsgemisch die höherreaktive Isocyanatkomponente zudosiert.

Gegenstand der Erfindung sind auch die nach diesem Verfahren erhaltenen Mischtrimerisate.

Gegenstand der Erfindung ist schließlich auch die Verwendung der nach diesem Verfahren erhaltenen Mischtrimerisate mit freien Isocyanatgruppen, gegebenenfalls in mit Blockierungsmittel für organische Polyisocyanate blockierter Form, als Ausgangsmaterial zur Herstellung von Polyurethankunststoffen nach dem Isocyanat-Polyadditionsverfahren.

Beim erfindungsgemäßen Verfahren werden als Ausgangsmaterialien zwei Isocyanatkomponenten eingesetzt, die jeweils aus mindestens einem Isocyanat bestehen, wobei die Isocyanatgruppen der beiden Komponenten eine im Sinne der Trimerisierungsreaktion unterschiedliche Reaktivität aufweisen. Diese erfindungswesentliche Bedingung ist dann erfüllt, wenn gleiche Gewichtsteile der jeweiligen Isocyanatkomponenten zum Erreichen des selben Trimerisierungsgrades bei gleicher Starttemperatur, gleicher Reaktionszeit und unter Verwendung eines 1 : 1-Komplexes aus Kaliumacetat und 18-Krone-6 sich bezüglich der hierfür erforderlichen Katalysatorkonzentration mindestens um den Faktor 2 unterscheiden. In zwei einfachen Vorversuchen kann somit leicht überprüft werden, ob diese erfindungswesentliche Bedingung erfüllt ist.

Unter »Trimerisierungsgrad« ist hierbei der Prozentsatz der in den Ausgangsisocyanaten vorliegenden Isocyanatgruppen zu verstehen, die bei der Trimerisierungsreaktion unter Isocyanuratbildung abreagiert haben. Falls beim erfindungsgemäßen Verfahren als Isocyanatkomponente a) mit Isocyanatgruppen einer vergleichsweise niedrigen Reaktivität und/oder als Isocyanatkomponente b) mit Isocyanatgruppen einer vergleichsweise höheren Reaktivität Isocyanatgemische eingesetzt werden, muß die genannte Bedingung bezüglich der unterschiedlichen Reaktivität zwischen dem Isocyanat der Komponente a) mit der höchsten Reaktivität und dem Isocyanat der Komponente b) mit der niedrigsten Reaktivität erfüllt sein.

Die genannte Bedingung bezüglich der unterschiedlichen Reaktivität ist im allgemeinen bei Verwendung von Isocyanaten mit aliphatischen und/oder cycloaliphatischen Isocyanatgruppen einerseits und Isocyanaten mit aromatisch gebundenen Isocyanatgruppen andererseits stets erfüllt. Derartige Kombinationen werden daher beim erfindungsgemäßen Verfahren bevorzugt eingesetzt. Es handelt sich bei diesen Isocyanaten vorzugsweise um solche der Formel

$$Q(NCO)_n$$

in welcher

Q für einen aliphatischen Kohlenwasserstoffrest mit 1—18 Kohlenstoffatomen, einen cycloaliphatischen Kohlenwasserstoffrest mit 3—15 Kohlenstoffatomen, einen araliphatischen Kohlenwasserstoffrest mit 7—20 Kohlenstoffatomen oder einen, gegebenenfalls methylsubstituierten, aromatischen Kohlenwasserstoffrest mit insgesamt 6 bis 15 Kohlenstoffatomen steht und

n für eine ganze Zahl von 1 bis 4 steht.

Die araliphatischen Isocyanate stellen hierbei ebenfalls Isocyanate mit aliphatisch gebundenen Isocyanatgruppen dar.

Typische Beispiele für Isocyanate mit aliphatisch gebundenen Isocyanatgruppen sind Methylisocyanat, Ethylisocyanat, Propylisocyanat, n-Butylisocyanat, tert.-Butylisocyanat, Isohexylisocyanat, Dodecylisocyanat, Oleylisocyanat, Stearylisocyanat, Cyclohexylisocyanat, Benzylisocyanat, $\alpha$-Methylbenzylisocyanat, Phenylethylisocyanat, Triphenylmethylisocyanat, Tetramethylendiisocyanat, Hexamethylendiisocyanat, Nonamethylendiisocyanat, 5-Methyl-nonamethylendiisocyanat, 1,4-Xylylendiisocyanat, 1,3-Xylylendiisocyanat, $\omega,\omega'$-Diisocyanato-1,4-diethylbenzol, Cyclobutan-1,3-diisocyanat,

Cyclohexan-1,4-diisocyanat, Cyclohexan-1,3-diisocyanat, Di-(4-isocyanatocyclohexyl)-methan, 1-Isocyanato-3-(isocyanatomethyl)-3,5,5-trimethylcyclohexan, 2,2,4-Trimethyl-hexamethylendiisocyanat, 2,4,4-Trimethylhexamethylen-diisocyanat, 1,12-Dodecandiisocyanat, 1,6,11-Triisocyanatoundecan, 2,4- oder 2,6-Hexahydrotoluylendiisocyanat, 1,3- oder 1,4-Hexahydrophenylendiisocyanat, 1,3-Butadienylen-1,4-diisocyanat oder 2,4'-Perhydrodiphenylmethandiisocyanat.

Typische Beispiele für erfindungsgemäß geeignete Isocyanate mit aromatisch gebundenen Isocyanatgruppen sind Phenylisocyanat, 4-, 3- oder 2-Methylphenylisocyanat, 1-Naphthylisocyanat, 1,3- oder 1,4-Phenylendiisocyanat, 2,4- oder 2,6-Toluylendiisocyanat, 2,4'-Diphenylmethandiisocyanat, 4,4'-Diphenylmethandiisocyanat, Naphthylen-1,5-diisocyanat, 4,4',4''-Triisocyanatotriphenyl-methan, 2,4,6-Triisocyanato-toluol oder Polyphenyl-polymethylen-polyisocyanate, wie sie durch Anilin-Formaldehyd-Kondensation und anschließende Phosgenierung hergestellt werden (»Roh-MDI«).

Neben diesen, der obigen allgemeinen Formel entsprechenden Ausgangsisocyanaten können beim erfindungsgemäßen Verfahren jedoch auch durch Heteroatome unterbrochene und/oder substituierte Isocyanate mit aliphatischen oder aromatisch gebundenen Isocyanatgruppen bzw. heteroaromatische Isocyanate eingesetzt werden. Beispiele hierfür sind 2-Chlorethylisocyanat, 2-Cyanethylisocyanat, 6-Chlorhexylisocyanat, Isocyanatoessigsäureethylester, Propyletherpropylisocyanat, 3-Methoxypropylisocyanat, 2-(2-Methoxyethoxy)-ethylisocyanat, 3,6,9-Trioxadecylisocyant, Allylisocyanat, 1,3-Pentadienylisocyanat, 1,2-Ethylendiisocyanat, $\omega,\omega'$-Diisocyanato-n-propylether; Lysinmethylesterdiisocyanat, Lysin-(2-isocyanatoethylester)-diisocyant, 4- oder 3-Chlorphenylisocyanat, 3,4-, 2,3-, 2,4-, 2,5- oder 2,6-Dichlorphenylisocyanat, 2,4,5- oder 2,4,6-Trichlorphenylisocyanat, 4-, 3- oder 2-Nitrophenylisocyanat, 2,5-Dichlor-4-nitrophenylisocyanat, 2,4-Dichlor-6-nitrophenylisocyanat, 2,6-Dichlor-4-nitrophenylisocyanat, 3- oder 2-Chlor-4-nitrophenylisocyanat, 2-Chlor-3-nitrophenylisocyanat, 4-Chlor-3- oder 2-nitrophenylisocyanat, 5-Chlor-2-nitrophenylisocyanat, 4-Trichlor-, tribrom- oder trifluormethylphenylisocyanat, 3-Chlor-4-trifluormethylphenylisocyanat, 3-Trifluormethylphenylisocyanat, 3-Chlor-4-methylphenylisocyanat, 4-Methoxyphenylisocyanat, 4-Trichlor-, tribrom oder trifluormethoxyphenylisocyanat, 4-Methylmercapto-phenylisocyanat, 4-Trichlor-, tribrom- oder trifluormethylmercapto-phenylisocyanat, 4-(1,1,2-Trifluor-2-chlorethanmercapto)-phenylisocyanat, 4-(1,1,2-Trifluor-2-chlorethoxy)-phenylisocyanat, 4-Phenoxyphenylisocyanat, 4-(4'-Chlorphenoxy)-phenylisocyanat, 4-(3'-Trifluormethylphenoxy)-phenylisocyanat, Tris-(4-isocyanatophenyl)-orthophosphorsäureester, 2-Isocyanato-3,4,5,6-tetrachlorpyridin, 2-Isocyanato-4,5,6-trichlorpyrimidin, 6-Isocyanato-2,4,5-trichlorpyrimidin, 6-Isocyanato-4,5-dichlor-2-trichlormethylpyrimidin, 2-Isocyanato-3,5,6-trichlorpyridazin, 4,6-Dichlor-s-triazinylisocyanat, 6-Chlor-s-triazinylendiisocyanat.

Das erfindungsgemäße Verfahren ist nicht auf den Einsatz von Kombinationen von Isocyanaten mit aliphatisch bzw. cycloaliphatisch gebundenen Isocyanatgruppen einerseits und Isocyanaten mit aromatisch gebundenen Isocyanatgruppen andererseits beschränkt. Vielmehr kann die allein erfindungswesentliche Bedingung sehr unterschiedlichen Reaktivität auch dann erfüllt sein, wenn beispielsweise Isocyanate mit aliphatisch oder cycloaliphatisch gebundenen Isocyanatgruppen einerseits mit Isocyanaten mit heteroaromatisch gebundenen Isocyanatgruppen andererseits kombiniert werden. Die erfindungswesentliche Bedingung der unterschiedlichen Reaktivität kann jedoch auch dann erfüllt sein, wenn nur Isocyanatgruppen mit aliphatisch und/oder cycloaliphatisch gebundenen Isocyanatgruppen oder nur Isocyanate mit aromatisch bzw. heteroaromatisch gebundenen Isocyanatgruppen zum Einsatz gelangen, falls die unterschiedliche Reaktivität der Isocyanatgruppen wegen des Vorliegens sterischer Effekte (Kombinationen von sterisch ungehinderten Isocyanaten mit Isocyanaten mit sterisch gehinderten Isocyanatgruppen), durch aktivierende bzw. desaktivierende Substituenten (z. B. Kombinationen von Elektronen anziehenden Substituenten wie z. B. Trifluormethylgruppen, Nitrogruppen oder Halogenatome aufweisenden aromatischen Isocyanaten mit unsubstituierten, alkyl- bzw- alkoxysubstituierten aromatischen Isocyanaten) und/oder durch die Bindungsart der Isocyanatgruppen (z. B. Kombinationen von aliphatischen Isocyanaten, deren Isocyanatgruppen an primäre Kohlenstoffatome gebunden sind mit aliphatischen Isocyanaten, deren Isocyanatgruppen an sekundäre oder tertiäre Kohlenstoffatome gebunden sind) gewährleistet ist. So erfüllen beispielsweise auch Kombinationen wie Phenylisocyanat/p-Trifluormethoxyphenylisocyanat, p-Trifluormethylphenylisocyanat/p-Trifluormethylmercaptophenylisocyanat, Allylisocyanat/Butylisocyanat, Allylisocyanat/Hexamethylendiisocyanat, Hexamethylendiisocyanat/Di-(4-isocyanatocyclohexyl)-methan, Hexamethylendiisocyanat/3,6,9-Trioxadecylisocyanat, 1,3-Pentadienylisocyanat/1-Isocyanato-3-isocyanatomethyl-3,5,5-trimethylcyclohexan und 6-Chlor-s-triazinylendiisocyanat/2,4-Toluylendiisocyanat die erfindungswesentliche Bedingung bezüglich der unterschiedlichen Reaktivität (das erstgenannte ist jeweils das reaktivere Isocyanat).

Besonders bevorzugt werden beim erfindungsgemäßen Verfahren als Isocyanate mit vergleichsweise niedriger Reaktivität aliphatische oder cycloaliphatische Diisocyanate der beispielhaft genannten Art, die neben den Isocyanatgruppen keine Heteroatome aufweisen, und als Isocyanate mit vergleichsweise höherer Reaktivität gegebenenfalls Methyl-substituierte aromatische Diisocyanate, die, von den Isocyanatgruppen abgesehen, keine weiteren Heteroatome aufweisen, eingesetzt.

Als Trimerisierungskatalysatoren kommen alle beliebigen Trimerisierungskatalysatoren des Standes der Technik in Betracht, wie sie beispielsweise in den oben genannten Publikationen veröffentlicht sind, vorausgesetzt der Katalysator ist aktiv genug, um das reaktionsträgere Isocyanat bei unterhalb

100° C liegenden Temperaturen zu trimerisieren. Vorzugsweise werden phosphinfreie Katalysatoren verwendet.

Bei der Herstellung von Isocyanatgruppen-freien Mischtrimerisaten aus Monoisocyanaten spielt es auch keine Rolle, ob sich der Katalysator thermisch oder durch Zugabe eines Katalysatorgifts desaktivieren läßt, da eine Unterbrechung der Trimerisierungsreaktion hier nicht erforderlich ist. Bei der Herstellung von Isocyanatgruppen aufweisenden Trimerisaten unter Verwendung von Polyisocyanaten, insbesondere von Verwendung von Diisocyanaten muß die Trimerisierungsreaktion in an sich bekannter Weise durch thermische Zersetzung und/oder durch Zugabe eines Katalysatorengifts beim jeweils gewünschten Trimerisierungsgrad unterbrochen werden. Hier ist selbstverständlich die Verwendung entsprechender Katalysatoren erforderlich.

Besonders gut geeignete Katalysatoren sind

a) 1 : 1-Komplexe von (i) basischen Natrium- oder Kaliumverbindungen und (ii) 1,4,7,10,13-Pentaoxacyclopentadecan (»15-Krone-5«) bzw. 1,4,7,10,13,16-Hexaoxacyclooxadecan (»18-Krone-6«). Hierbei sind unter »1 : 1-Komplexen« Komplexe von äquimolaren Mengen einer basischen Natrium- oder Kaliumverbindung mit 15-Krone-5 bzw. 18-Krone-6 zu verstehen. Die Komplexierung der Natriumverbindungen geschieht mit dem erstgenannten, die Komplexierung der Kaliumverbindungen mit dem letztgenannten cyclischen Polyether.

Als basische Natrium- oder Kaliumverbindungen kommen beliebige Verbindungen der genannten Alkali-Metalle in Betracht, deren wäßrige Lösung in einmolarer Konzentration einen pH-Wert von mindestens 7,5 aufweist.

Geeignete basische Verbindungen sind beispielsweise Natrium- oder Kaliumcarboxylate mit vorzugsweise 1 bis 12 Kohlenstoffatomen, -alkoholate mit vorzugsweise 1 bis 8 Kohlenstoffatomen, -phenolate mit vorzugsweise 6 bis 10 Kohlenstoffatomen, -carbonate, -hydroxide, -cyanate, Enolate oder -cyanide. Geeignete basische Natrium- oder Kaliumverbindungen sind z. B. die Formiate, Acetate, Propionate, 2-Ethylhexanoate, n-Dodecanoate, Caprylate, Methylate, Ethylate, Butylate, Hexylate, Phenolate, tert.-Butylphenolate, Carbonate, Hydroxide, Cyanate, Thiocyanate oder Cyanide der genannten Metalle oder aber auch beispielsweise Natrium- oder Kalium-N-methylacetamid. Zu den bevorzugten basischen Verbindungen gehören die genannten Carboxylate, Alkoholate, Phenolate, Carbonate, Hydroxide, Cyanate und Cyanide. Besonders bevorzugt sind einfache Carboxylate der genannten Alkali-Metalle mit 1 bis 4 Kohlenstoffatomen, insbesondere Kaliumacetat.

Die zur Komplexbildung eingesetzten cyclischen Polyether stellen bekannte Verbindungen dar. Ihre Herstellung kann beispielsweise gemäß G. Johns, C. J. Ransom, C. B. Reese, Synthesis (1976), Seite 515 erfolgen.

Die Herstellung der 1 : 1-Komplexe kann beispielsweise nach einer der nachstehenden Methoden erfolgen:

1. Die Herstellung erfolgt unter Verwendung des zu trimerisierenden Isocyanats bzw. seiner Lösung in einem geeigneten Lösungsmittel, welches auch als Reaktionsmedium bei der Durchführung des erfindungsgemäßen Verfahrens dienen kann, dergestalt, daß man den cyclischen Polyether in dem Isocyanat geringerer Reaktivität bzw. dessen Lösung auflöst und das Alkali-Metallsalz als Feststoff unter Komplexbildung und Auflösung einrührt.
2. Der cyclische Polyether wird in einem geeigneten Lösungsmittel aufgelöst und danach das Alkali-Metallsalz unter Komplexbildung und Auflösen zudosiert. Eventuelle Trübungen werden durch Filtration entfernt.
3. Es wird wie unter 2. beschrieben verfahren, jedoch unter Verwendung eines relativ leichtflüchtigen Lösungsmittels, welches im Anschluß an die Komplexbildung abgezogen wird, so daß der Komplex als fester Rückstand anfällt, der anschließend in einem anderen Lösungsmittel und/oder in dem Isocyanat geringerer Reaktivität gelöst wird.

Bei der Herstellung der 1 : 1-Komplexe werden vorzugsweise die Komponenten (i) und (ii) in äquimolaren Mengen eingesetzt. Selbstverständlich wäre es auch möglich, in anderen Mengenverhältnissen zu arbeiten, was jedoch zur Folge hätte, daß entweder die basische Alkali-Metallverbindung oder der cyclische Polyether im Überschuß vorliegt. Wie leicht einzusehen ist, wäre eine derartige Verfahrensweise wenig zweckmäßig, da der jeweilige Überschuß keine oder nur eine vergleichsweise schlechte katalytische Wirkung aufweisen würde. Bei der Herstellung von Lösungen der 1 : 1-Komplexe werden die Komponenten (i) und (ii) im allgemeinen in solchen Mengen eingesetzt, daß 0,4 bis 40, vorzugsweise 0,8 bis 20gew.-%ige Lösungen der Komplexe vorliegen. Es ist gerade einer der Hauptvorteile der Katalysatoren, daß sie in derartigen, vergleichsweise hohen Konzentrationen in den nachstehend beispielhaft genannten Lösungsmitteln löslich sind.

Lösungsmittel, die, wie oben aufgezeigt, gegebenenfalls auch als Reaktionsmedium zur Herstellung der Komplexe verwendet werden können, sind insbesondere die in der Polyurethan-Lack-Technologie üblichen polaren, physiologisch weitgehend unbedenklichen Lösungsmittel eines bei Normaldruck zwischen 50° C und 350° C liegenden Siedepunkts oder alkoholische Hydroxylgruppen aufweisende,

bei Raumtemperatur flüssige Verbindungen eines zwischen 32 und 250, vorzugsweise 46 und 162 liegenden Molekulargewichts. Selbstverständlich können auch beliebige Gemische derartiger Lösungsmittel eingesetzt werden. Beispiele geeigneter Lösungsmittel der genannten Art sind: Ethylacetat, Butylacetat, Ethylglykolacetat, Aceton, Methylethylketon, Methylisobutylketon, Cyclohexanon, Methoxyhexanon oder auch Chlorkohlenwasserstoffe wie z. B. Chloroform oder Chlorbenzol. Mit Verschnittmitteln wie z. B. Toluol, Xylol und höheren Aromaten besteht nur eine begrenzte Löslichkeit. Höhere Zusätze derartiger Lösungsmittel können zu Trübungen und Ausfällungen führen. Beispiele von als Lösungsmittel geeigneten Verbindungen mit alkoholischen Hydroxylgruppen sind: Methanol, Ethanol, Isopropanol, Ethylenglykolacetat, Ethylenglykol, Diethylenglykol, Ethylenglykol-monoethylether, Glycerin oder Trimethylolpropan. Da die 1 : 1-Komplexe in derartigen, Hydroxylgruppen aufweisenden Verbindungen im allgemeinen eine sehr gute Löslichkeit aufweisen, so daß die alkoholischen Lösungsmittel nur in sehr geringen Mengen eingesetzt werden müssen, stört ihre Anwesenheit bei der Durchführung des erfindungsgemäßen Verfahrens nicht.

Neben diesen genannten Lösungsmitteln können auch höher siedende Lösungsmittel wie z. B. die üblichen Weichmacher wie Dibutylphthalat, Butylbenzylphthalat oder Phosphorsäureester wie Tricresylphosphat Verwendung finden.

b) Komplexe aus (i) basischen Alkalimetallverbindungen der genannten Art und (iii) acyclischen organischen Verbindungen, die

mindestens 5 Alkylenoxideinheiten der Formel $-R-O-$ ($R=C_1-C_4$-Alkylen) in Form einer oder mehrerer Polyetherketten mit jeweils mindestens 3 Alkylenoxideinheiten,
einen Gehalt an innerhalb mindestens einer Polyetherkette angeordneten Alkylenoxideinheiten der genannten Art von insgesamt mindestens 40 Gew.-% und
ein Molekulargewicht von mindestens 238 aufweisen.

Bei der Katalysatorkomponente (iii) handelt es sich hierbei im Unterschied zu den oben genannten cyclischen Verbindungen (ii) um acylische organische Verbindungen, die die obengenannten Kriterien erfüllen, insbesondere um solche, die
mindestens 5 Alkylenoxideinheiten der genannten Art, vorzugsweise Ethylenoxid- und gegebenenfalls Propylenoxideinheiten in Form einer oder mehrerer Polyetherketten mit jeweils mindestens 3, vorzugsweise jeweils mindestens 5 Alkylenoxideinheiten,
einen Gehalt an innerhalb von Polyetherketten eingebauten Alkylenoxideinheiten der genannten Art von mindestens 55 Gew.-%, wobei mindestens 50%, vorzugsweise mindestens 80%, aller vorliegender Alkylenoxideinheiten Ethylenoxideinheiten darstellen, und
ein Molekulargewicht von 238—3000, besonders bevorzugt von 266—1000 aufweisen.

Typische Beispiele für geeignete Katalysatorkomponenten (iii) sind diesen Definitionen entsprechende 1- bis 3-wertige Polyetheralkohole, wie sie in an sich bekannter Weise durch Alkoxylierung, insbesondere Ethoxylierung geeigneter Startermoleküle wie einwertigen Alkoholen wie Methanol, Ethanol, n- oder i-Propanol, n-, i-, sec.- oder tert.-Butanol von Wasser oder von mindestens zweiwertigen Startermolekülen wie Ethylenglykol, Propandiol-(1,2), Propandiol-(1,3), Butan-, Pentan- oder Hexandiolen, Glycerin, Trimethylolethan oder Trimethylolpropan erhalten werden können. Geeignet sind auch solche Polyether der beispielhaft genannten Art, deren endständige(n) Hydroxylgruppe(n) beispielsweise durch eine Alkylierung, Acylierung und/oder Urethanisierung verschlüsselt wurden, so daß keine Endgruppen mehr vorliegen, deren Reaktivität gegenüber Isocyanatgruppen mit der Reaktivität von Hydroxylgruppen vergleichbar ist, oder Derivate der genannten Polyetheralkohole, die durch Umsetzung mit Kettenverlängerungsmitteln, beispielsweise Diisocyanaten wie Tetramethylendiisocyanat, Hexamethylendiisocanat oder 2,4-Diisocyanatotoluol kettenverlängert worden sind, soweit die so erhaltenen Derivate den oben gemachten Definitionen entsprechen.

Der Verschlüsselung der endständigen Hydroxylgruppe(n) der oben beispielhaft genannten Polyetheralkohole durch Alkylierung geschieht beispielsweise durch Umsetzung der Polyetheralkohole mit Alkylierungsmitteln wie z. B. Dimethylsulfat, $C_1-C_4$-Alkyl-Halogeniden oder Benzylhalogenid; die Verschlüsselung durch eine Acylierungsreaktion durch Umsetzung mit Acylierungsmitteln wie z. B. Essigsäureanhydrid, Acetylchlorid oder Benzoylchlorid; die Verschlüsselung durch Urethanisierung durch Umsetzung mit einwertigen Isocyanaten wie z. B. Methyl-, Ethyl-, Hexyl- oder Phenylisocyanat. Durch Umsetzung der beispielhaft genannten Polyetheralkohole mit Aldehyden wie Formaldehyd, Acetaldehyd oder Benzaldehyd im Sinne einer Acetalisierung werden gegebenenfalls substituierte Methylenoxideinheiten in die Polyether eingeführt.

Zur Herstellung dieser als Trimerisierungskatalysatoren einzusetzenden Komplexe werden die beispielhaft genannten Komponenten (i) und (iii) bzw. beliebige ihrer Gemische in solchen Mengenverhältnissen miteinander umgesetzt, daß auf jedes Grammäquivalent an basischer Metallverbindung (i) 0,8 bis 2,0, vorzugsweise 1,0 bis 1,5 Mol an, obigen Definitionen entsprechenden, Verbindungen (iii) zur Verfügung stehen. Falls bei der Herstellung der Komplexe Alkoxylierungsprodukte der beispielhaft genannten Startermoleküle bzw. deren Derivate eingesetzt werden, die wegen der statistisch verlaufenden Alkoxylierungsreaktion Gemische darstellen, die neben den obigen Definitionen entsprechenden Verbindungen (iii) auch diesen Definitionen, beispielsweise wegen eines zu geringen Gehalts an Alkylenoxideinheiten, nicht entsprechenden Verbindungen enthalten, muß die Menge solcher Gemische selbstverständlich so bemessen werden, daß obige Mengenverhältnisse der Komponenten (i) und (iii) eingehalten werden. Die Umsetzung zwischen den Komponenten (i) und (iii), d. h. die Komplexbildung, erfolgt im allgemeinen spontan bei einer Temperatur von ca. 10 bis 60° C, insbesondere wenn miteinander verträgliche Komponenten (i) und (iii) in Abwesenheit von Lösungsmitteln eingesetzt werden. Die Bildung der Komplexe kann jedoch auch in Gegenwart von Lösungsmitteln bzw. Lösungsmittelgemischen der oben beispielhaft genannten Art erfolgen.

Falls die Komponenten (i) und (iii) miteinander verträglich und/oder in dem verwendeten Lösungsmittel leicht löslich sind, können die Gemische aus (i) und (iii) bzw. deren Lösungen unmittelbar nach ihrer Herstellung als Trimerisierungskatalysatoren eingesetzt werden. Es ist in diesem Fall auch möglich, die Komponenten (i) und (iii) getrennt dem zu trimerisierenden Isocyanat vergleichsweise niedriger Reaktivität zuzudosieren, obwohl dies weniger bevorzugt ist. Falls die Komponenten (i) und (iii) jedoch miteinander unverträglich und im verwendeten Lösungsmittel nicht oder nur schwer löslich sind, ist es oft zweckmäßig die zunächst gebildeten Suspensionen während ca. 15 bis 360 Minuten inneralb des genannten Temperaturbereichs mechanisch zu durchmischen und einen etwa noch verbleibenden Niederschlag abzufiltrieren. Die dann vorliegende Lösung des Komplexes kann als solche eingesetzt oder im Vakuum eingeengt werden. Der zurückbleibende, lösungsmittelfreie Komplex kann schließlich in anderen, bevorzugt aprotischen Lösungsmitteln, gelöst werden. So können beispielsweise bei Raumtemperatur ca. 0,02 molare »Lösungen von Kaliumacetat« in Toluol hergestellt werden.

Bei der Durchführung des erfindungsgemäßen Verfahrens muß nicht auf eine strikte Abwesenheit von Alkohol in Lösungsmitteln für die Komplexe bzw. von Hydroxylgruppen aufweisenden Komplexbildnern (iii) geachtet werden, da die allenfalls zur Debatte stehenden äußerst geringen Mengen an gegenüber Isocyanatgruppen reaktionsfähigen Gruppen im Vergleich zu den Isocyanatgruppen des zu trimerisierenden Ausgangspolyisocyanats vernachlässigbar sind.

Die Durchführung des erfindungsgemäßen Verfahrens kann lösungsmittelfrei oder in Gegenwart von beliebigen organischen Lösungsmitteln, die gegenüber Isocyanatgruppen inert sind und für die Ausgangsisocyanate Löser darstellen, der oben bei der Beschreibung der bevorzugten Katalysatoren genannten Art erfolgen.

Bei der Durchführung des erfindungsgemäßen Verfahrens wird das Mengenverhältnis der Isocyanatkomponente a) mit Isocyanatgruppen vergleichsweise erniedrigter Reaktivität zur Isocyanatkomponente b) mit Isocyanatgruppen vergleichsweise erhöhter Reaktivität so bemessen, daß ein NCO-Äquivalentverhältnis a) : b) von 1 : 1 bis 10 : 1, vorzugsweise 2 : 1 bis 8 : 1, vorliegt. Die Isocyanatkomponente a) wird bei der Durchführung des erfindungsgemäßen Verfahrens bei 10 bis 100° C, vorzugsweise 20 bis 50° C, vorgelegt und dann mit der zum Start der Trimerisierung gerade ausreichenden Menge Katalysator versetzt. Im Falle der besonders bevorzugten komplexierten Alkaliverbindungen liegt diese Menge im allgemeinen bei 0,0005 bis 0,5, vorzugsweise 0,001 bis 0,1 Gew.-%, bezogen auf Komplexierungsmittel-freie Alkaliverbindungen einerseits und Isocyanatkomponente a) andererseits. Sobald die eotherme Trimerisierungsreaktion zu einem Trimerisierungsgrad von 0,1 bis 8%, vorzugsweise 0,5 bis 5% geführt hat, gibt man die reaktive Isocyanatkomponente b) kontinuierlich hinzu und zwar so, daß die Reaktion zwischen 30 und 100° C, bevorzugt 50 und 70° C, stets exotherm bleibt. Die Reaktionstemperatur wird über die Zugabegeschwindigkeit und geeignete Kühlmaßnahmen geregelt. Nach Beendigung der Zugabe läßt man die Mischung bis zum Erreichen des gewünschten Trimerisierungsgrades weiterrühren und stoppt dann die Umsetzung durch Inhibierung des Katalysators ab.

Wie bereits dargelegt ist jedoch eine Abstoppung der Trimerisierungsreaktion im Falle der Herstellung von Isocyanatgruppen-freien Trimerisaten aus Monoisocyanaten nicht erforderlich. Die Desaktivierung des Katalysators erfolgt im Falle von thermisch labilen Trimerisierungskatalysatoren beispielsweise im Falle der Verwendung von Hydroxyalkyl-Substituenten aufweisenden quartärneren Ammoniumhydroxiden thermisch durch einen kurzen Temperaturschock, vorzugsweise jedoch durch Zugabe eines Katalysatorengifts. Im Falle der Verwendung der besonders bevorzugten komplexierten Alkaliverbindungen eignen sich insbesondere Säurechloride wie Benzoylchlorid, Acetylchlorid, Bernsteinsäuredichlorid, Terephthalsäuredichlorid, 2,5-Dichlorbenzoesäurechlorid, Phosphortrichlorid oder Thionylchlorid bzw. starke Säuren wie p-Toluolsulfonsäure, Nonafluorbutansulfonsäure oder Phosphorsäure sowie Carbaminsäurechloride, wie sie durch Addition von HCl an Isocyanate gebildet werden können, die unter Neutralisation der basischen Alkalimetallverbindungen die erfindungswesentlichen Katalysatoren desaktivieren als Katalysatorengift.

Im Falle der Herstellung von Isocyanatgruppen aufweisenden Trimerisaten erfolgt die Abstoppung der Reaktion im allgemeinen bevor ein Trimerisierungsgrad von 50%, vorzugsweise bevor ein Trimerisierungsgrad von 30%, bezogen auf die Gesamtmenge der eingesetzten Isocyanate, erreicht wurde. In dem dann vorliegenden Reaktionsgemisch liegen im allgemeinen keinerlei Isocyanate b) mit Isocyanatgruppen vergleichsweise erhöhter Reaktivität mehr vor, so daß im Gemisch neben dem Verfahrensprodukt lediglich die Isocyanatkomponente a) mit Isocyanatgruppen vergleichsweise erniedrigter Reaktivität vorliegt. Das überschüssige Ausgangsisocyanat kann dann gewünschtenfalls durch Extraktion oder vorzugsweise durch Destillation, insbesondere im Dünnschichtverdampfer, vom Verfahrensprodukt abgetrennt werden.

Die erfindungsgemäßen Verfahrensprodukte mit freien Isocyanatgruppen stellen wertvolle Ausgangsmaterialien zur Herstellung von Polyurethankunststoffen nach dem Isocyanat-Polyadditionsverfahren dar. Hierzu können sie in an sich bekannter Weise sowohl in freier als auch in mit Blockierungsmittel für Isocyanatgruppen blockierter Form eingesetzt werden. Die Isocyanatgruppen-freien erfindungsgemäßen Verfahrensprodukte auf Basis von Monoisocyanate stellen neue Verbindungen dar, die als biologisch wirksame Substanzen Verwendung finden können.

In den nachfolgenden Beispielen beziehen sich alle Prozentangaben auf Gewichtsprozente. Die Identifizierung und Charakterisierung der beschriebenen Verfahrensprodukte erfolgt durch eine kombinierte Anwendung der Massenspektrometrie, Hoch-Druck-Flüssigkeits-Chromatographie, Gaschromatographie und Gelchromatographie.

## Beispiel 1

### Prüfung auf unterschiedliche Reaktivität

a) 100 g 2,4-Toluylendiisocyanat (NCO-Gehalt 48,25%) werden bei 25° C mit 7,4 mg (0,02 mmol) eines 1 : 1-Komplexes aus Kaliumacetat und 18-Krone-6, gelöst in 1 ml 2-Ethylhexanol, versetzt. Nach 45 Minuten und einer Wärmetönung von 90° C beträgt der NCO-Gehalt 38,4%; dies entspricht einem Trimerisierungsgrad von 20%.

b) 100 g Hexamethylendiisocyanat (NCO-Gehalt 49,96%) werden bei 25° C mit 185 mg (0,51 mmol) eines 1 : 1-Komplexes aus Kaliumacetat und 18-Krone-6, gelöst in 1 ml 2-Ethylhexanol, versetzt. Nach 45 Minuten und einer Wärmetönung von 65° C beträgt der NCO-Gehalt 40,0%; dies entspricht einem Trimerisierungsgrad von 20%.

Die erfindungswesentliche Bedingung der unterschiedlichen Reaktivität ist erfüllt, da sich die erforderliche Katalysatorkonzentration um den Faktor 25 unterscheidet.

## Beispiel 2

### (Vergleich)

348 g 2,4-Toluylendiisocyanat (TDI) und 168 g Hexamethylendiisocyanat (HDI) werden bei 25° C unter Feuchtigkeitsausschluß mit 0,15 mmol eines 1 : 1-Komplexes aus Kaliumacetat und 18-Krone-6 versetzt. Der Reaktionsansatz erwärmt sich innerhalb von 35 Minuten auf 58° C, danach hält man die Temperatur durch zusätzliche Heizung auf 50° C. Nach 4 Std. Rühren ist der NCO-Gehalt auf 38,7% abgesunken und man stoppt die Umsetzung durch Zugabe von 0,15 mmol Benzoylchlorid ab. Das Produkt ist nicht dünnschichtbar, da in der Kolonne sofortige Kristallisation eintritt. Im Reaktionsprodukt findet man als monomeres Isocyanurat ausschließlich Molmasse 522 (reines TDI-Trimerisat). Der Anteil der monomeren Isocyanate enthält 47,3% TDI und 52,7% HDI.

## Beispiel 3

### (Vergleich)

168 g HDI und 87 g TDI werden bei 25° C mit 0,5 ml einer 0,5molaren Lösung von Kaliumacetat in Polyethylenglykol (mittl. Mol-Gew. 370) versetzt. Der Reaktionsansatz erwärmt sich innerhalb von 25 Min. auf 69° C. Nach 32 Minuten beträgt die Temperatur 63° C und der NCO-Gehalt ist auf 38,0% abgesunken. Anschließend stoppt man die Umsetzung durch Zugabe von 0,25 mmol Benzoylchlorid ab. Das Reaktionsprodukt verhält sich wie das aus Vergleichsbeispiel 1a; es ist nicht dünnschichtbar. Destilliert man bei 0,5 mbar am Rotationsverdampfer (Badtemperatur 200° C), so erhält man 97 g eines kristallinen Rückstandes, welcher als monomeres Isocyanurat hauptsächlich nur reines TDI-Trimerisat (Mol-Gew. 522) enthält. Im Destillat (156 g) beträgt der Anteil des HDI 95%.

Die beiden Vergleichsbeispiele 2 und 3 zeigen, daß unabhängig vom Mischungsverhältnis die Trimerisation eines Gemisches aus HDI und TDI im wesentlichen reines TDI-Trimerisat ergibt; lediglich in den höhermolekularen Oligomeren sind ca. 10 bis 30% HDI mit eingebaut.

Beispiel 4

(erfindungsgemäß)

500 g Hexamethylendiisocyanat (HDI) werden unter Rühren und Feuchtigkeitsausschluß bei 25°C mit 1,0 ml einer 0,5molaren Lösung von Kaliumacetat in Polyethylenglykol (mittl. Mol-Gew. 370; Katalysatorkonzentration = 0,01% Kaliumacetat auf HDI) versetzt. Nach 15 Min. beträgt der NCO-Gehalt 48,8% (Trimerisierungsgrad = 2,3%) und die Reaktionstemperatur 40°C. Nun tropft man kontinuierlich innerhalb von 40 Min. 100 g 2,4-Toluylendiisocyanat (TDI) zu, wobei die Temperatur langsam auf 52°C ansteigt. Nach dem Zutropfen beträgt der NCO-Gehalt 41,6%. Man stoppt die Umsetzung durch Zugabe von 0,25 mmol Hexamethylendicarbaminsäurechlorid (HDI × 2 HCl) ab. Nach Dünnschichtdestillation (Vakuum 0,8 mbar, Umlauftemperatur 180°C; freies TDI im Destillat 4%) erhält man 148 g eines leicht gelblich gefärbten Produktes, das abgemischt mit Butylacetat (60% Feststoffgehalt) einen NCO-Gehalt von 11,7%, eine Viskosität (25°C) von 505 mPas und einen Monomerengehalt HDI/TDI < 0,2% besitzt. Der Anteil an TDI im Trimerisat beträgt 48%, wobei die Homotrimerisate der Molmassen 504 (3 HDI) und 522 (3 TDI) jedoch zusammen nur 5 bis 10% ausmachen. Man findet bei den Monoisocyanuraten sowohl Molmasse 510 (1 TDI/2 HDI) als auch Molmasse 516 (2 TDI/1 HDI).

Beispiel 5

(erfindungsgemäß)

504 g Hexamethylendiisocyanat (HDI) werden unter Rühren bei 25°C mit 5 ml einer 0,2molaren Lösung von Kaliumacetat/18-Krone-6 (1 : 1) in Diethylenglykolmonomethylether (=0,02% Kaliumacetat auf HDI) versetzt. Nach 4 Minuten beträgt die Reaktionstemperatur 30°C. Nun tropft man kontinuierlich innerhalb von 45 Min. 174 g 2,4-Toluylendiisocyanat (TDI) zu, wobei die Temperatur durch gelegentliche Kühlung zwischen 50 und 70°C gehalten wird. Nach dem Zutropfen beträgt der NCO-Gehalt 37,6%. Nach weiteren 5 Min. ist der NCO-Gehalt auf 37% abgesunken und man stoppt die Umsetzung durch Zugabe von 1 mmol Nonafluorbutansulfonsäure ab. Nach Dünnschichtdestillation (freies TDI im Destillat 0,6%) erhält man 287 g eines leicht gelblichen Harzes mit 17,4% NCO-Gehalt, einem Gehalt an monomerem HDI von 0,19% und an monomerem TDI von 0,1%. Der TDI-Anteil im Produkt beträgt 57%. Für die Monoisocyanurate findet man wiederum alle theoretisch möglichen Molmassen: 504, 510, 516 und 522, wobei der Anteil der Homotrimerisate unter 20% liegt. Das Gelchromatogramm zeigt, daß neben den Monoisocyanuraten (ca. 30%) auch höherverzweigte Oligomere wie Diisocyanurate (ca. 20%), Tri-, Tetra- und Pentaisocyanurate (ca. 30%) sowie ca. 20% miteinander verknüpfte Isocyanurate mit einer Molmasse > 2500 vorliegen.

Beispiel 6

(erfindungsgemäß)

2016 g Hexamethylendiisocyanat (HDI) werden unter Rühren und Feuchtigkeitsausschluß bei 25°C mit 7,0 ml einer 0,5molaren Lösung von Kaliumacetat in Polyethylenglykol (mittleres Mol-Gew. 370; Katalysatorkonzentration = 0,017% Kaliumacetat auf HDI) versetzt. Nach 10 Min. beträgt die Reaktionstemperatur 35°C. Nun tropft man kontinuierlich innerhalb von 50 Min. 696 g 2,4-Toluylendiisocyanat (TDI) zu, wobei die Temperatur durch gelegentliches Kühlen zwischen 40 und 50°C gehalten wird. Nach dem Zutropfen beträgt der NCO-Gehalt 36,0% und die Temperatur 45°C. Man rührt 10 Min. weiter, bis der NCO-Gehalt auf 35,2% abgefallen ist und stoppt dann durch Zugabe von 3 mmol Benzoylchlorid ab. Nach Dünnschichtdestillation erhält man 1362 g Destillat, in dem sich 0,3% TDI befinden, sowie 1297 g Trimerisat mit einem NCO-Gehalt von 18,0% und einem Gehalt an freiem HDI/TDI von 0,15%. Der Anteil an eingebautem TDI liegt bei 51%. TDI-Homotrimerisat wird nur in Spuren gebildet.

Beispiel 7

(Vergleich)

30 g Phenylisocyanat und 105 g Hexamethylendiisocyanat (HDI) werden bei 25°C mit 0,6 ml einer 0,5molaren Lösung von Kaliumacetat in Polyethylenglykol (mittleres Mol-Gew. 370; 0,03% Kaliumacetat auf HDI) versetzt. Die Lösung erwärmt sich innerhalb von 30 Min. auf 70°C und hat dann einen NCO-Gehalt von 29%. Nach weiteren 30 Min. ist die Temperatur wieder auf 25°C abgefallen und ein kristallines Produkt hat sich abgeschieden. Der NCO-Gehalt ist nicht weiter gefallen. Nach Verdünnen mit Toluol, Filtration und Umkristallisation aus Eisessig erhält man 60% Phenylisocyanurat, Schmp. 274°C.

Beispiel 8

(erfindungsgemäß)

252 g Hexamethylendiisocyanat (HDI) werden unter Rühren bei 25°C mit 1,25 ml einer 0,5molaren Lösung von Kaliumacetat in Polyethylenglykol (mittl. Mol-Gew. 370; Katalysatorkonzentration = 0,025% Kaliumacetat auf HDI) versetzt. Nach 30 Min. beträgt die Reaktionstemperatur 40° C. Nun tropft man kontinuierlich innerhalb von 135 Min. 60 g Phenylisocyanat zu, wobei die Temperatur durch Kühlung zwischen 60 und 80° C gehalten wird. Nach dem Zutropfen beträgt der NCO-Gehalt der klaren Lösung 19,6% und die Umsetzung wird durch Zugabe von 0,6 mmol Nonafluorbutansulfonsäure abgebrochen. Nach Dünnschichtdestillation (freies Phenylisocyanat im Destillat <0,1%) erhält man ein zähviskoses, leicht gelbliches Produkt mit einem NCO-Gehalt von 13,1% und einem Gehalt an freiem HDI von 0,15%. Das Produkt setzt sich zusammen aus den Monoisocyanuraten der Molmassen 406 (2 PI/1 HDI), 455 (1 PI/2 HDI), 504 (3 HDI), sowie wenig 357 (3 PI).

Außerdem erhält es neben höhermolekularen Oligomeren Trimerisierungsprodukte der allgemeinen Formel

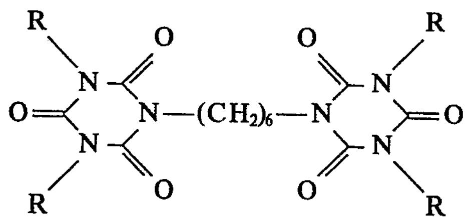

R = Phenyl, $-(CH_2)_6-NCO$

wobei alle theoretischen möglichen Molmassen (644, 693, 742, 791 und 840) vorhanden sind.

Beispiel 9

(erfindungsgemäß)

504 g Hexamethylendiisocyanat (HDI) werden unter Rühren und Feuchtigkeitsausschluß bei 25°C mit 1,5 ml einer 0,5molaren Lösung von Kaliumacetat in Polyethylenglykol (mittl. Mol-Gew. 370; Katalysatorkonzentration = 0,015% Kaliumacetat auf HDI) versetzt. Nach 20 Min. beträgt die Reaktionstemperatur 40° C. Nun tropft man kontinuierlich innerhalb von 35 Min. aufgeschmolzenes 4,4'-Diisocyanatodiphenylmethan (MDI) zu, wobei die Temperatur durch gelegentliche Kühlung zwischen 50 und 70° C gehalten wird. Nach dem Zutropfen läßt man noch 10 Min. weiterrühren, bis der NCO-Gehalt 28,0% beträgt. Dann stoppt man die Umsetzung durch Zugabe von 0,7 mmol Nonafluorbutansulfonsäure ab. Nach Dünnschichtdestillation (freies MDI im Destillat 0,2%) erhält man 430 g eines farblosen zähen Produktes mit einem NCO-Gehalt von 16,0%, einem Gehalt an freiem HDI von 0,18% und an freiem MDI von 0,4%. Im Produkt findet sich das Mischtrimerisat der Formel

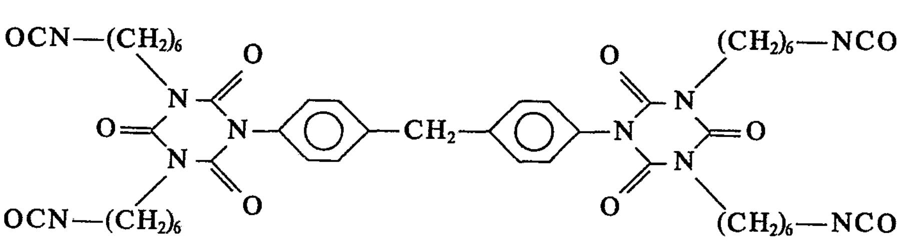

neben den Monoisocyanuraten der Molmasse 504 (3 HDI), 586 (2 HDI/1 MDI) und 668 (1 HDI/2 MDI) sowie höhermolekularen Oligomeren. Der Anteil an eingebautem MDI beträgt 29%.

Beispiel 10

(Vergleich)

a) Zu 10 g Phenylisocyanat, gelöst in 15 ml Toluol, gibt man 5 ml einer 0,02molaren Lösung von Kaliumacetat/18-Krone-6 (1 : 1) in Butylacetat. Innerhalb von 2 Minuten erwärmt sich der Reaktionsansatz auf 100° C und alles Phenylisocyanat hat trimerisiert.

b) Zu 10 g p-Trifluormethoxyphenylisocyanat, gelöst in 7,5 ml Toluol, gibt man 12,5 ml einer 0,02molaren Lösung von Kaliumacetat/18-Krone-6 (1 : 1) in Butylacetat. Innerhalb von 2 Stunden ist alles Isocyanat trimerisiert; die Temperaturerhöhung beträgt 53° C.

c) Zu 11,5 g p-Trifluormethoxyphenylisocyanat und 5,0 g Phenylisocyanat, die in 15 ml Toluol gelöst sind, gibt man bei 25° C 5 ml einer 0,02molaren Lösung von Kaliumacetat/18-Krone-6 (1 : 1) in Butylacetat. Innerhalb von 6 Stunden und 30 Min. hat alles Isocyanat abreagiert, wobei zwischenzeitlich eine Temperaturerhöhung auf 40° C eintritt. Nach Filtration des Feststoffes erhält man 7,7 g (67%) Trifluormethoxyphenylisocyanurat. Im Filtrat findet man Phenylisocyanurat und 1,3-(Di-p-trifluormethoxyphenyl)-5-phenyl-1,3,5-triazin-2,4,6-[1H,3H,5H]-trion; 3,5-Diphenyl-1-(p-trifluormethoxyphenyl)-1,3,5-triazin-2,4,6-(1H,3H,5H)-trion kann nicht nachgewiesen werden.

Beispiel 11

(erfindungsgemäß)

11,5 g p-Trifluormethoxyphenylisocyanat werden in 10 ml Toluol gelöst und bei 25° C mit 10 ml einer 0,02molaren Lösung von Kaliumacetat/18-Krone-6 (1 : 1) in Butylacetat versetzt. Nachdem sich der Reaktionsansatz auf 30° C erwärmt hat, gibt man innerhalb von 5 Minuten 5,0 g Phenylisocyanat hinzu und rührt bis sich alles Isocyanat umgesetzt hat. Nach säulenchromatographischer Trennung erhält man die beiden Mischisocyanurate 1,3-(Di-p-trifluormethoxyphenyl)-5-phenyl-1,3,5-triazin-2,4,6(1H,3H,5H)-trion und 3,5-Diphenyl-1-(p-trifluormethoxyphenyl)-1,3,5-triazin-2,4,6(1H,3H,5H)-trion in 73%iger Ausbeute.

**Patentansprüche**

1. Verfahren zur Herstellung von Mischtrimerisaten durch zumindest teilweise Trimerisierung der Isocyanatgruppen von mindestens zwei organischen Isocyanaten einer im Sinne der Trimerisierungsreaktion unterschiedlichen Reaktivität in Gegenwart von, die Trimerisierung von Isocyanatgruppen unter Isocyanuratbildung beschleunigenden, Katalysatoren und gegebenenfalls Abbruch der Trimerisierungsreaktion beim jeweils erwünschten Trimerisierungsgrad durch thermische Zersetzung des Trimerisierungskatalysators und/oder durch Zugabe eines Katalysatorengifts, dadurch gekennzeichnet, daß man die weniger reaktive Isocyanatkomponente vorlegt, anschließend in Gegenwart des Katalysators unter Trimerisierung von mindestens 0,1% der Isocyanatgruppen anpolymerisiert und schließlich zu dem so erhaltenen Reaktionsgemisch die höherreaktive Isocyanatkomponente zudosiert.

2. Verfahren gemäß Anspruch 1, dadurch gekennzeichnet, daß man die höherreaktive Isocyanatkomponente zudosiert, sobald 0,5 bis 5% der Isocyanatgruppe der vorgelegten Isocyanatkomponente in trimerisierter Form vorlegen.

3. Verfahren gemäß Anspruch 1 und 2, dadurch gekennzeichnet, daß man als Trimerisierungskatalysatoren phosphinfreie Trimerisierungskatalysatoren verwendet.

4. Verfahren gemäß Anspruch 1 bis 3, dadurch gekennzeichnet, daß man als weniger reaktive Isocyanatkomponente organische Isocyanate mit aliphatisch und/oder cycloaliphatisch gebundenen Isocyanatgruppen und als höherreaktive Isocyanatkomponente organische Isocyanate mit aromatisch gebundenen Isocyanatgruppen verwendet.

5. Verfahren gemäß Anspruch 1 bis 4, dadurch gekennzeichnet, daß man als Ausgangsisocyanate organische Diisocyanate verwendet.

6. Verfahren gemäß Anspruch 1 bis 5, dadurch gekennzeichnet, daß man die Trimerisierungsreaktion durch thermische Vernichtung des Trimerisierungskatalysators und/oder durch Zugabe eines Katalysatorgifts abbricht, bevor 50% der insgesamt im Reaktionsgemisch vorliegenden Isocyanatgruppen in trimerisierter Form vorliegen.

7. Verfahren gemäß Anspruch 6, dadurch gekennzeichnet, daß man nach dem Abbruch der Trimerisierungsreaktion das im Reaktionsgemisch vorliegende nicht umgesetzte Ausgangsisocyanat durch Destillation oder Extraktion abtrennt.

8. Gemäß Anspruch 1 bis 7 erhaltene Mischtrimerisate.

9. Verwendung der gemäß Anspruch 1 bis 7 erhaltenen, freie Isocyanatgruppen aufweisenden Mischtrimerisate, gegebenenfalls in mit Blockierungsmittel für organische Polyisocyanate blockierter Form, als Ausgangsmaterial zur Herstellung von Polyurethankunststoffen nach dem Isocyanat-Polyadditionsverfahren.

**Claims**

1. Process for the preparation of mixed trimers by at least partial trimerisation of the isocyanate groups of at least two organic isocyanates having different reactivities with respect to the trimerisation reaction, in the presence of catalysts which accelerate the trimerisation of isocyanate groups with

the formation of isocyanurates and, optionally, termination of the trimerisation reaction at the degree of trimerisation required in each case by thermal decomposition of the trimerisation catalyst and/or by the addition of a catalyst poison, characterised in that the less reactive isocyanate component is initially introduced, then polymerisation is commenced in the presence of the catalyst with trimerisation of at least 0.1% of the isocyanate groups and finally the more reactive isocyanate component is metered into the reaction mixture thus obtained.

2. Process according to Claim 1, characterised in that the more reactive isocyanate component is metered in as soon as 0.5 to 5% of the isocyanate group of the initially introduced isocyanate component are in a trimerised form.

3. Process according to Claim 1 and 2, characterised in that phosphine-free trimerisation catalysts are used as the trimerisation catalysts.

4. Process according to Claim 1 to 3, characterised in that organic isocyanates containing aliphatically and/or cycloaliphatically bound isocyanate groups are used as the less reactive isocyanate component and organic isocyanates containing aromatically bound isocyanate groups are used as the more reactive isocyanate component.

5. Process according to Claim 1 to 4, characterised in that organic diisocyanates are used as the starting isocyanates.

6. Process according to Claim 1 to 5, characterised in that the trimerisation reaction is terminated by thermal destruction of the trimerisation catalyst and/or by the addition of a catalyst poison, before 50% of the total isocyanate groups present in the reaction mixture are in a trimerised form.

7. Process according to Claim 6, characterised in that after the termination of the trimerisation reaction the unreacted starting isocyanate present in the reaction mixture is separated off by distillation or extraction.

8. Mixed trimers obtained according to Claim 1 to 7.

9. Use of the mixed trimers containing free isocyanate groups obtained according to Claim 1 to 7, optionally in a form blocked with blocking agents for organic polyisocyanates, as a starting material for the production of polyurethane plastics by the isocyanate polyaddition process.

## Revendications

1. Procédé d'obtention de produits mixtes de trimérisation par trimérisation au moins partielle des groupes isocyanate d'au moins deux isocyanates organiques de réactivité différente dans le sens de la réaction de trimérisation, en présence de catalyseurs accélérant la trimérisation de groupes isocyanate avec formation d'isocyanurate et, le cas échéant, interruption de la réaction de trimérisation à chaque degré désiré de trimérisation, par décomposition thermique du catalyseur de trimérisation et/ou par addition d'un poison pour le catalyseur, caractérisé en ce qu'on introduit au préalable le composant isocyanate le moins réactif, puis on amorce la polymérisation en présence du catalyseur avec trimérisation d'au moins 0,1% des groupes isocyanate et on ajoute finalement au mélange réactionnel ainsi obtenu le composant isocyanate de plus grande réactivité.

2. Procédé suivant la revendication 1, caractérisé en ce qu'on ajoute le composant isocyanate de plus grande réactivité aussitôt qu'une proportion de 0,5 à 5% du groupe isocyanate du composant isocyanate préalablement introduit se trouve sous la forme trimérisée.

3. Procédé suivant les revendications 1 et 2, caractérisé en ce qu'on utilise comme catalyseurs de trimérisation des catalyseurs de trimérisation ne contenant pas de phosphine.

4. Procédé suivant les revendications 1 à 3, caractérisé en ce qu'on utilise comme composant isocyanate moins réactif, des isocyanates organiques à groupes isocyanate en liaison aliphatique et/ou en liaison cycloaliphatique et, comme composant isocyanate à réactivité supérieure, des isocyanates organiques à groupes isocyanate en liaison aromatique.

5. Procédé suivant les revendications 1 à 4, caractérisé en ce qu'on utilise des diisocyanates organiques comme isocyanates de départ.

6. Procédé suivant les revendications 1 à 5, caractérisé en ce que la réaction de trimérisation est interrompue par destruction thermique du catalyseur de trimérisation et/ou par addition d'un poison pour le catalyseur avant que 50% des groupes isocyanate existant au total dans le mélange réactionnel se trouvent sous la forme trimérisée.

7. Procédé suivant la revendication 6, caractérisé en ce que l'isocyanate de départ n'ayant pas réagi se trouvant dans le mélange réactionnel est séparé par distillation ou par extraction après interruption de la réaction de trimérisation.

8. Des produits mixtes de trimérisation obtenus conformément aux revendications 1 à 7.

9. Utilisation des produits mixtes de trimérisation obtenus suivant les revendications 1 à 7, présentant des groupes isocyanate libres, le cas échéant sous la forme bloquée avec des agents de blocage pour polyisocyanates organiques, comme matière de départ pour la production de matières premières du type polyuréthanne par le procédé de polyaddition d'isocyanate.